# EUROPÄISCHE PATENTSCHRIFT

(11) **EP 3 914 317 B1**
(45) Veröffentlichungstag und Bekanntmachung des Hinweises auf die Patenterteilung: **06.11.2024**
(21) Anmeldenummer: 20701962.1
(22) Anmeldetag: 22.01.2020
(51) Int. Cl.: A61M 1/36, G01F 1/36, G01F 1/72, G01F 15/00, G01F 23/30

(54) **VERFAHREN ZUM BEFÜLLEN EINES MEMBRANFILTERS**
METHOD FOR FILLING A MEMBRANE FILTER
PROCÉDÉ POUR LE REMPLISSAGE D'UN FILTRE À MEMBRANE

(30) Priorität: 22.01.2019 DE 102019101542
(43) Veröffentlichungstag der Anmeldung: 01.12.2021
(73) Patentinhaber: Fresenius Medical Care Deutschland GmbH, 61352 Bad Homburg (DE)
(72) Erfinder: NOACK, Joachim, 97616 Bad Neustadt (DE)
(74) Vertreter: Behr, Wolfgang
(86) Internationale Anmeldenummer: PCT/EP2020/051487
(87) Internationale Veröffentlichungsnummer: WO 2020/152209

(56) Entgegenhaltungen:
- EP-A1- 2 662 101
- DE-C1- 10 011 208
- US-A1- 2014 217 027

## Beschreibung

Die vorliegende Erfindung betrifft ein Verfahren zum Befüllen eines Membranfilters eines Blutbehandlungssystems, wobei das Blutbehandlungssystem wenigstens einen Membranfilter, insbesondere einen Hohlfasermembranfilter, mit einer ersten und einer zweiten Kammer, welche durch eine Membran semipermeabel getrennt sind, mindestens einen ersten Teilkreislauf und mindestens einen zweiten Teilkreislauf aufweist, wobei die erste Kamer des Membranfilters im ersten Teilkreislauf und die zweite Kammer des Membranfilters im zweiten Teilkreislauf angeordnet ist, und wobei die Befüllung der ersten Kammer des Membranfilters über den ersten Teilkreislauf mit Flüssigkeit erfolgt, während die zweite Kammer noch mit Luft gefüllt ist. Weiterhin umfasst die vorliegende Erfindung eine entsprechende Blutbehandlungsmaschine und eine Blutbehandlungssystem.

Ein Füllverfahren ist aus der DE 10 2011 102 492 A1 bekannt. Dabei erfolgt die Befüllung der ersten Kammer des Membranfilters über den ersten Teilkreislauf so, dass zumindest über eine Teilphase der Befüllung Luft von dem ersten Teilkreislauf in den zweiten Teilkreislauf verdrängt wird.

Die DE 10 2015 009 886 A1 zeigt weiterhin ein Verfahren, bei welchem nach der Befüllung der ersten Kammer des Membranfilters über den ersten Teilkreislauf ein Überdruck gegenüber der zweiten Kammer an der ersten Kammer angelegt wird.

Aus der WO 2008/125893 A1 ist es weiterhin bekannt, zur Befüllung des ersten und des zweiten Teilkreislaufs die venöse und die arterielle Leitung des zweiten Teilkreislaufs mit einem stromabwärts des Membranfilters gelegenen Abschnitt des ersten Teilkreislaufs zu verbinden, und stromabwärts des Membranfilters und stromabwärts der Verbindungsstellen mit der venösen und arteriellen Leitung im ersten Teilkreislauf angeordnete Pumpen in Abhängigkeit von einem Druck im ersten und im zweiten Teilkreislauf anzusteuern. Die Ansteuerung einer stromaufwärts des Membranfilters angeordneten Pumpe erfolgt dagegen volumengesteuert.

DE 100 11 208 C1 zeig ein weiteres Verfahren zur Befüllung eines Schlauchsets einer Dialysemaschine.

Ein Verfahren gemäß dem Oberbegriff von Anspruch 1 ist aus US 2014/0217027 A1 bekannt.

Im Sinne dieser Beschreibung bedeuten der Begriff "stromaufwärts" und stromabwärts" die Flussrichtung wie die Flüssigkeiten bei Betrieb während einer Behandlung die Flüssigkeiten die Leitungen durchströmen. Beispielsweise kann sich das daran erkennbar sein, dass sich stromabwärts des Membranfilters ein Blutleckdetektor angeordnet sein kann. Dieser kann dazu geeignet sein, Blut, das während der Behandlung vom zweiten Teilkreislauf in den ersten Teilkreislauf durch die Membran übertritt, zu detektieren. Stromabwärts kann eine Luftabscheidekammer angeordnet sein. Bezüglich des bei Betrieb mit Blut gefüllten Teilkreislaufs kann "stromaufwärts" den Leitungsabschnitt bezeichnen, in dem eine Blutpumpe und/oder eine Zugabestelle für Heparin angeordnet ist.

Aufgabe der vorliegenden Erfindung ist es, ein Verfahren und eine Behandlungsmaschine zur Verfügung zu stellen, welche eine zuverlässige Befüllung des Membranfilters erlauben.

Diese Aufgabe wird durch ein Verfahren gemäß Anspruch 1 und eine Behandlungsmaschine gemäß Anspruch 14 gelöst.

Ausgestaltungen der vorliegenden Erfindung sind Gegenstand der Unteransprüche.

Die vorliegende Erfindung umfasst ein Verfahren zum Befüllen eines Membranfilters eines Blutbehandlungssystems, wobei das Blutbehandlungssystem wenigstens eine Blutbehandlungsmaschine, einen Membranfilter, insbesondere einen Hohlfasermembranfilter, mit einer ersten und einer zweiten Kammer, welche durch eine Membran semipermeabel getrennt sind, und mindestens einen ersten Teilkreislauf und mindestens einen zweiten Teilkreislauf aufweist, wobei die erste Kammer des Membranfilters im ersten Teilkreislauf und die zweite Kammer des Membranfilters im zweiten Teilkreislauf angeordnet ist, wobei die Befüllung der ersten Kammer des Membranfilters über den ersten Teilkreislauf mit Flüssigkeit erfolgt, während die zweite Kammer noch mit Luft gefüllt ist, und wobei stromaufwärts des Membranfilters eine Pumpe im ersten Teilkreislauf angeordnet ist. Dabei ist vorgesehen, dass die Ansteuerung der Pumpe zur Befüllung der ersten Kammer des Membranfilters über den ersten Teilkreislauf in Abhängigkeit von einem im ersten Teilkreislauf gemessenen Druck und/oder dem Transmembrandruck erfolgt.

Durch die Ansteuerung der Pumpe in Abhängigkeit des Drucks wird eine noch zuverlässigere Befüllung des Membranfilters ermöglicht. So kann in machen Ausführungsbeispielen das Verbleiben von Luftinseln innerhalb des Membranfilters weiter verringert werden. In manchen Ausführungsbeispielen kann der Befüllungszustand erfasst werden.

In einer bevorzugten Ausgestaltung handelt es sich bei dem ersten Teilkreislauf um den Dialysatkreislauf und/oder bei dem zweiten Teilkreislauf um einen extrakorporalen Blutkreislauf. Insbesondere betrifft die vorliegende Erfindung daher die Befüllung der Dialysatkammer eines Dialysators.

In einer möglichen Ausgestaltung der vorliegenden Erfindung wird während der Befüllung der ersten Kammer des Membranfilters mit Flüssigkeit in der ersten Kammer vorhandene Luft über die Membran in die zweite Kammer des Membranfilters und damit in den zweiten Teilkreislauf verdrängt.

In einer möglichen Ausgestaltung der vorliegenden Erfindung erfolgt die Befüllung der ersten Kammer des Membranfilters über den ersten Teilkreislauf von oben nach unten. Bevorzugt erfolgt die Befüllung der ersten Kammer des Membranfilters über den ersten Teilkreislauf mit der gleichen Flussrichtung, mit welcher der Membranfilter auch während der Blutbehandlung durchströmt wird.

In einer bevorzugten Ausgestaltung erfolgt die Befüllung der beiden Kammern des Membranfilters ohne eine zwischengeschaltete Drehung des Membranfilters.

Der Membranfilter wird in einer möglichen Ausgestaltung der vorliegenden Erfindung während der Blutbehandlung im ersten und um zweiten Teilkreislauf im Gegenstrom betrieben. Wird daher die erste Kammer des Membranfilters über den ersten Teilkreislauf, insbesondere im Dialysatkreislauf, von oben nach unten befüllt, kann die zweite Kammer des Membranfilters über den zweiten Teilkreislauf, insbesondere im Blutkreislauf, mit der auch während der Blutbehandlung eingesetzten Flussrichtung von unten nach oben befüllt und/oder gespült werden, ohne den Membranfilter zu drehen. Hierdurch kann eine sichere Entlüftung der zweiten Kammer des Membranfilters erreicht werden.

In einer möglichen Ausgestaltung der vorliegenden Erfindung wird der durch die Pumpe erzeugte, zur Befüllung eingesetzte Volumenstrom und/oder der zeitliche Verlauf und/oder die Dauer der Befüllung durch die Pumpe in Abhängigkeit von einem im ersten und/oder zweiten Teilkreislauf gemessenen Druck angesteuert.

Gemäß der vorliegenden Erfindung wird ein Druck im ersten Teilkreislauf und/oder ein Transmembrandruck über die Membran des Membranfilters gemessen, wobei die Ansteuerung der Pumpe zur Befüllung der ersten Kammer des Membranfilters über den ersten Teilkreislauf in Abhängigkeit von dem Druck im ersten Teilkreislauf und/oder dem Transmembrandruck erfolgt. Insbesondere kann der Druck stromaufwärts und/oder stromabwärts der ersten Kammer im ersten Teilkreislauf gemessen und die Befüllung der ersten Kammer des Membranfilters über den ersten Teilkreislauf in Abhängigkeit von diesem Druck erfolgen.

Die Bestimmung des Transmembrandrucks kann anhand eines Drucks, gemessen mittels eines Drucksensors am zweiten Teilkreislauf, und eines Drucks, gemessen mittels eines weiteren Drucksensors an ersten Teilkreislauf, erfolgen. Der Transmembrandruck kann mittels dieser zweier Druckwerte und eines dritten Druckwertes, gemessen mittels eines dritten Drucksensors am ersten Teilkreislauf, bestimmt werden, wobei der weitere Drucksensor stromaufwärts und der dritte Drucksensor stromabwärts des Membranfilters angeordnet sein kann. Die Bestimmung kann erfolgen, indem der Mittelwert der mittels des weiteren Drucksensors und des dritten Drucksensors gemessenen Drücke gebildet wird und daran der Druck im zweiten Teilkreislauf abgezogen wird oder der Mittelwert kann an dem Drucks im zweiten Teilkreislauf abgezogen werden.

Die vorliegende Erfindung beruht auf der Erkenntnis, dass einige Membranfilter, insbesondere einige Dialysatoren, sehr empfindlich bezüglich des Drucks über die Membran sind, solange die zweite Kammer noch nicht mit Flüssigkeit gefüllt ist. Steht in dieser Situation ein bestimmter Transmembrandruck über einen bestimmten Zeitraum an dem Membranfilter an, so tritt Flüssigkeit von der ersten Kammer, insbesondere der Dialysatkammer, in die zweite Kammer, insbesondere die Blutkammer über und diese kann später nicht mehr ordentlich gefüllt werden. Dabei ist zu berücksichtigen, dass im Dialysator dort, wo Flüssigkeit die Kapillaren gefüllt hat, keine Luft mehr durch die Kapillare der Faserwandung hindurchtreten kann. Umgekehrt wird ein gewisser Transmembrandruck jedoch benötigt, um die Luft effektiv von der ersten Kammer über die Membran in die zweite Kammer zu verdrängen.

Da der Transmembrandruck entscheidend von dem Druck im ersten Teilkreislauf und insbesondere von dem Druck stromaufwärts der ersten Kammer bestimmt wird, kann alternativ zum Transmembrandruck auf der Druck im ersten Teilkreislauf und insbesondere der Druck stromaufwärts der ersten Kammer gemessen und zur Ansteuerung herangezogen bzw. begrenzt werden.

In einer möglichen Ausgestaltung wird die Befüllung so angesteuert, dass der Druck im ersten Teilkreislauf und/oder der Transmembrandruck einen ersten Schwellwert nicht übersteigt.

Bevorzugt wird eine Förderrate der Befüllung verringert, sobald der Druck im ersten Teilkreislauf und/oder der Transmembrandruck einen ersten Schwellwert übersteigt. Die Verringerung der Befüllung kann auch ein komplettes Stoppen der Befüllung umfassen. Insbesondere kann ein Flüssigkeitszufluss so lange verringert und insbesondere gestoppt werden, bis der Druck im ersten Teilkreislauf und/oder der Transmembrandruck wieder unterhalb des ersten oder eines zweiten Schwellwertes liegt.

Erfindungsgemäß wird eine Förderrate der Pumpe bei der Befüllung verringert oder die Pumpe gestoppt, sobald der Druck im ersten Teilkreislauf und/oder der Transmembrandruck einen ersten Schwellwert übersteigt, wobei der erste Schwellwert beispielsweise mindestens 50 mBar beträgt, beispielsweise mindestens 100 mBar, oder beispielsweise mindestens 200 mBar.

Erfindungsgemäß wird eine Förderrate der Pumpe bei der Befüllung erhöht oder die Pumpe wieder gestartet wird, sobald der Druck im ersten Teilkreislauf und/oder der Transmembrandruck einen zweiten Schwellwert unterschreitet, wobei der zweite Schwellwert beispielsweise mindestens 50 mBar beträgt, oder beispielsweise mindestens 100 mBar, oder beispielsweise mindestens 150 mBar.

Bevorzugt ist ein Absolutwert des ersten Schwellwerts größer ist als ein Absolutwert des zweiten Schwellwertes. Wenn die beiden Schwellwerte gleich groß sind, könnte dies dazu führen, dass die Pumpe ständig anlaufen und stoppen würde. Durch unterschiedliche Schwellwerte wird dies verhindert.

In einer möglichen Ausgestaltung wird die Pumpe so angesteuert, dass in der ersten Kammer des Membranfilters zumindest zeitweise ein Überdruck entsteht, insbesondere ein Druck von beispielsweise mindestens 50 mbar, oder beispielsweise mindestens 100 mBar, oder beispielsweise mindestens 200 mBar.

In einer möglichen Ausgestaltung wird die Pumpe zur Befüllung der ersten Kammer des Membranfilters volumengesteuert angesteuert, wobei die volumengesteuerte Ansteuerung gestoppt oder der Volumenstrom verringert wird, sobald der Druck im ersten Teilkreislauf und/oder der Transmembrandruck einen ersten Schwellwert überschreitet, wobei der erste Schwellwert beispielsweise mindestens 50 mBar beträgt, oder beispielsweise mindestens 100 mBar, oder beispielsweise mindestens 200 mBar.

In einer möglichen Ausgestaltung wird die volumengesteuerte Ansteuerung wiederaufgenommen oder der Volumenstrom vergrößert, sobald der Druck im ersten Teilkreislauf und/oder der Transmembrandruck einen zweiten Schwellwert unterschreitet, wobei der zweite Schwellwert beispielsweise mindestens 50 mBar beträgt, oder beispielsweise mindestens 100 mBar, oder beispielsweise mindestens 150 mBar.

Bevorzugt ist ein Absolutwert des ersten Schwellwerts größer ist als ein Absolutwert des zweiten Schwellwertes.

Durch die erfindungsgemäße Druck- bzw. Flussbegrenzung kann sichergestellt werden, dass keine Luftpolster im Faserbündel des Membranfilters eingeschlossen werden, die anschließend nicht mehr abgeschieden werden können. Weiterhin stellt die erfindungsgemäße Ansteuerung jedoch sicher, dass ein ausreichender Druck an der ersten Kammer anliegt, um dort befindliche Luft über die Membran in die zweite Kammer zu verdrängen.

In einer möglichen Ausgestaltung wird die erste Kammer des Membranfilters mit einem pulsatilen Volumenstrom befüllt, durch welchen in der ersten Kammer des Membranfilters Druckspitzen erzeugt werden. Die Druckspitzen und der darauf jeweils folgende Druckabfall entstehen dabei im Rhythmus des pulsatilen Volumenstroms.

Die Pumpe wirkt bevorzugt mit einer Bilanzkammeranordnung zusammen, welche durch die Pumpe mit Flüssigkeit beaufschlagt wird und deren Umschaltungen zu einem pulsatilen Volumenstrom führen.

In einer möglichen Ausgestaltung wird während der Befüllung der ersten Kammer des Membranfilters zumindest zeitweise keine Flüssigkeit aus dem System abgeführt. Dies ermöglicht eine schnellere Füllung des ersten Teilkreislaufs.

Insbesondere kann eine Fluidverbindung eines stromabwärts der ersten Kammer im ersten Teilkreislauf angeordneten Sekundärluftabscheiders mit dem Abfluss während der Befüllung geschlossen und/oder eine dort angeordnete Pumpe nicht betrieben werden. Insbesondere kann dies während der druckgesteuerten Befüllung erfolgen, insbesondere während der gesamten druckgesteuerten Befüllung.

In einer möglichen Ausgestaltung wird während der Befüllung der ersten Kammer des Membranfilters eine stromabwärts des Membranfilters im ersten Dialysatkreislauf angeordnete Pumpe zumindest zeitweise nicht betrieben. Insbesondere kann zumindest zeitweise keine stromabwärts des Membranfilters im ersten Dialysatkreislauf angeordnete Pumpe betrieben werden. Insbesondere kann die Pumpe oder Pumpen stromabwärts eines im ersten Teilkreislauf angeordneten Sekundärluftabscheiders angeordnet sein. Bevorzugt wird oder werden die Pumpe oder Pumpen während der gesamten druckgesteuerten Befüllung nicht betrieben.

In einer weiteren Variante wird der Druck im zweiten Teilkreislauf gemessen, wobei die Befüllung der ersten Kammer des Membranfilters über den ersten Teilkreislauf und insbesondere die Ansteuerung der Pumpe in Abhängigkeit von dem Druck im zweiten Teilkreislauf erfolgt.

Die Messung des Drucks im zweiten Teilkreislauf kann dabei in einer ersten Ausgestaltung der vorliegenden Erfindung Teil einer Messung des Transmembrandrucks sein, wie dies oben bereits beschrieben wurde.

Alternativ oder zusätzlich kann die Befüllung der ersten Kammer des Membranfilters über den ersten Teilkreislauf auch in Abhängigkeit von dem absoluten Druck und/oder Druckänderungen im zweiten Teilkreislauf erfolgen.

Diese Verfahrensführung beruht auf der Erkenntnis, dass der Füllzustand der ersten Kammer des Membranfilters durch Messung des Drucks im zweiten Teilkreislauf bestimmt werden kann. Insbesondere macht sich die vorliegende Erfindung hierbei zunutze, dass sich bei einem Übertritt von Luft aus der ersten Kammer durch die Membran in den zweiten Teilkreislauf der Druck im zweiten Teilkreislauf erhöht.

Wenn der zweite Teilkreislauf zur Atmosphäre hin offen ist, ergeben sich durch die dennoch vorhandenen Flusswiderstände dynamische Druckänderungen. Ist der zweite Teilkreislauf zur Atmosphäre geschlossen, ergeben sich auch statische Druckänderungen.

Insbesondere können daher dynamische und/oder statische Druckänderungen im zweiten Teilkreislauf erfasst und/oder überwacht werden.

In einer möglichen Ausgestaltung der vorliegenden Erfindung wird der Druck im zweiten Teilkreislauf im Hinblick auf Druckänderungen, insbesondere Druckschwankungen überwacht. Bevorzugt hängt dabei der zeitliche Ablauf der Befüllung der ersten Kammer von einer Detektion von Druckänderungen, insbesondere Druckschwankungen ab, und insbesondere von einem Zeitpunkt, an welchem Druckänderungen, insbesondere Druckschwankungen erfasst und/oder nicht mehr erfasst werden.

In einer möglichen Ausgestaltung der vorliegenden Erfindung wird eine komplette Befüllung der ersten Kammer des Membranfilters durch die Überwachung auf Druckänderungen, insbesondere Druckschwankungen erkannt, wobei bevorzugt auf eine komplette Befüllung geschlossen wird, wenn keine Druckänderungen, insbesondere Druckschwankungen mehr erfasst werden

In einer möglichen Ausgestaltung der vorliegenden Erfindung wird die Befüllung der ersten Kammer so lange fortgesetzt, wie Druckänderungen, insbesondere Druckschwankungen im zweiten Teilkreislauf erfasst werden. Weiterhin kann noch eine Mindestrestfüllphase durchgeführt werden, nachdem keine Druckänderungen, insbesondere Druckschwankungen mehr erfasst werden.

In einer möglichen Ausgestaltung der vorliegenden Erfindung wird die Befüllung der ersten Kammer so lange fortgesetzt, wie Luft in einem sekundären Luftabscheider des ersten Teilkreislaufs erfasst wird, wobei bevorzugt noch eine Mindestrestfüllphase durchgeführt wird, nachdem keine Luft mehr erfasst wird.

Ist daher die Membran aufgrund des verwendeten Materials schon von vornherein nicht luftdurchlässig, oder wurde diese schon komplett getränkt, bevor die Luft komplett aus der ersten Kammer des Membranfilters verdrängt wurde, so wird die Luft bei einer (weiteren) Befüllung der ersten Kammer des Membranfilters über den ersten Teilkreislauf in den stromabwärts des Membranfilters im ersten Teilkreislauf angeordneten Sekundärluftabscheider verdrängt. Durch die Überwachung des Sekundärluftabscheiders kann daher sichergestellt werden, dass keine Luft mehr aus der ersten Kammer des Membranfilters in den ersten Teilkreislauf gepumpt wird und daher auch bei dieser Konstellation eine komplette Füllung vorliegt.

Durch die Überwachung des Drucks im zweiten Teilkreislauf und/oder der Luft im Sekundärluftabscheider wird sichergestellt, dass die Membran am Ende dieses Schritts vollständig benetzt ist, also der Dialysator bestmöglich, in einer Ausführungsform ohne Drehen, gefüllt ist.

Die Mindestrestfüllphase dient dann als Sicherheitspuffer und zum Spülen des Membranfilters. Diese kann eine konstante Länge aufweisen.

Die Mindestrestfüllphase kann ein vorbestimmtes Pumpvolumen, eine vorbestimmte Zeit oder eine vorbestimmte Anzahl an Pumpschlägen und/oder Bilanzkammerumschaltungen umfassen.

Bevorzugt erfolgt die Befüllung der ersten Kammer des Membranfilters über den ersten Teilkreislauf mit einem pulsatilen Volumenstrom. Der pulsatile Volumenstrom kann mittels eine Membranpumpe, beispielsweise in Form einer Bilanzkammer, generiert werden. Insbesondere kann die Befüllung über mehrere Bilanzkammerumschaltungen erfolgen. Die Befüllung kann jedoch auch mit einem konstanten Volumenstrom erfolgen.

In einer möglichen Ausgestaltung der vorliegenden Erfindung wird der erste Teilkreislaufs zumindest während einer Startphase der Befüllung der ersten Kammer mit einem ersten Volumenstrom von maximal 800 ml/min, bevorzugt von maximal 500 ml/min befüllt. Diese Begrenzung kann dabei unabhängig von dem gemessenen Druck vorliegen. Nach der Startphase kann mit einem größeren maximalen Volumenstrom gearbeitet werden, wobei der Volumenstrom in Abhängigkeit von einem gemessenen Druck gesteuert werde kann.

In einer möglichen Ausgestaltung der vorliegenden Erfindung ist der zweite Teilkreislauf während der Befüllung der ersten Kammer des Membranfilters über den ersten Teilkreislauf zur Atmosphäre hin offen, d.h. er steht in Fluidverbindung zur Atmosphäre. Hierdurch wird der durch die Luft im zweiten Teilkreislauf erzeugte Gegendruck verringert und/oder abgebaut.

In einer möglichen Ausgestaltung der vorliegenden Erfindung steht der zweite Teilkreislauf während der Befüllung der ersten Kammer des Membranfilters über ein Bauteil, das einen Druckabfall erzeugt, mit der Atmosphäre in Fluidverbindung, Die Verbindung zur Atmosphäre erfolgt bevorzugt über einen Filter, insbesondere einen Hydrophob- und/oder Sterilfilter und/oder eine Drossel und/oder ein Ventil. Hierdurch verbleibt ein gewisser Widerstand gegen die Befüllung des zweiten Teilkreislaufs über die Membran mit der Luft aus der ersten Kammer, was zu Druckveränderungen durch den Befüllprozess führt.

In einer möglichen Ausgestaltung der vorliegenden Erfindung wird während der Befüllung der ersten Kammer des Membranfilters über den ersten Teilkreislauf Flüssigkeit und/oder Luft aus der ersten Kammer in einen Sekundärluftabscheider, welcher im ersten Teilkreislauf stromabwärts des Membranfilters angeordnet ist, verdrängt. Hierdurch wird die Befüllung der ersten Kammer und die Entfernung von Luft aus der ersten Kammer verbessert.

In einer möglichen Ausgestaltung steht der Sekundärluftabscheider zumindest zeitweise nicht mit einem Flüssigkeitsabfluss des Systems in Verbindung, insbesondere indem die entsprechende Fluidverbindung geschlossen oder eine Pumpe nicht betrieben wird. Hierdurch fließt keine Flüssigkeit aus dem System ab, so dass die Befüllung beschleunigt wird.

In einer möglichen Ausgestaltung der vorliegenden Erfindung steht der Sekundärluftabscheider, welcher im ersten Teilkreislauf stromabwärts des Membranfilters angeordnet ist, während der Befüllung der ersten Kammer des Membranfilters über den ersten Teilkreislauf zumindest zeitweise nicht mit der Atmosphäre in Verbindung. Je nach Ausgestaltung der Fluidkreisläufe kann dies zu einem schnelleren Anstieg des Transmembrandrucks bis auf einen gewünschten Wert und damit zu einem größeren Volumenstrom durch die Membran führen.

Beispielsweise wird während der gesamten druckgesteuerten Befüllung eine Verbindung mit der Atmosphäre nur dann hergestellt, wenn Luft im Sekundärluftabscheider erfasst wird.

Der Sekundärluftabscheider kann ein Gefäß aufweisen, mit einem Zufluss und einem Abfluss, vorzugsweise im unteren Bereich, beispielsweise im Boden oder im unteren Drittel, des Gefäßes, so dass sich Luft im oberen Bereich des Gefäßes sammelt und zu einer Absenkung des Flüssigkeitspegels in dem Gefäß bewirkt. Das Absinken oder Ansteigen des Pegels kann mittels eines Pegelsensors von der Steuerung der Blutbehandlunsgvorrichtung erkennbar sein.

In einer möglichen alternativen Ausgestaltung der vorliegenden Erfindung steht ein Sekundärluftabscheider, welcher im ersten Teilkreislauf stromabwärts des Membranfilters angeordnet ist, während der Befüllung der ersten Kammer des Membranfilters über den ersten Teilkreislauf durchgehend mit der Atmosphäre in Verbindung. Hierdurch kann Luft auch durch den ersten Teilkreislauf in Richtung Atmosphäre verdrängt werden. Die Verbindung zur Atmosphäre kann durch eine Öffnung im oberen Bereich des Gefäßes des Sekundärluftabscheiders vorliegen.

In einer möglichen Ausgestaltung der vorliegenden Erfindung wird der erste Teilkreislauf während der druckgesteuerten Befüllung zumindest zeitweise nicht bilanzierend befüllt.

Nicht-bilanzierende Füllung hat dabei die Bedeutung, dass nicht dasselbe Flüssigkeitsvolumen dem ersten Teilkreislauf zugeführt und entnommen wird. Dadurch wird im Ergebnis dem ersten Teilkreislauf insgesamt Flüssigkeit zugeführt. Bei einem bilanzierenden Füllen wird dasselbe Flüssigkeitsvolumen dem ersten Teilkreislauf zugeführt und entnommen. Dadurch kommt es vor allem zu einer Verschiebung der Flüssigkeit im ersten Teilkreislauf und mit dieser Verschiebung der Flüssigkeit auch zu einem Verschieben von Luft, die dann in dem Sekundärluftabscheider abgetrennt werden kann.

In einer möglichen Ausgestaltung der vorliegenden Erfindung startet die Befüllung der ersten Kammer des Membranfilters über den ersten Teilkreislauf ohne eine bilanzierende Phase. Alternativ oder zusätzlich erfolgt die Befüllung der ersten Kammer des Membranfilters über den ersten Teilkreislauf zumindest bis zu einer Detektion einer Befüllung nicht-bilanzierend. Die Detektion der Befüllung kann beispielsweise anhand des Pegelsensors des Sekundärluftabscheiders erfolgen.

In einer möglichen Ausgestaltung der vorliegenden Erfindung startet die Befüllung der ersten Kammer des Membranfilters über den ersten Teilkreislauf dagegen mit einer bilanzierenden Phase, auf welche eine nicht-bilanzierende Phase folgt. In der bilanzierenden Phase wird die Luft aus der ersten Kammer bevorzugt in einen stromabwärts des Membranfilters im ersten Teilkreislauf angeordneten Sekundärluftabscheider verschoben.

Der Befüllung der ersten Kammer des Membranfilters geht bevorzugt eine Befüllung des ersten Teilkreislaufs voraus. Die Befüllung der ersten Kammer des Membranfilters startet daher bevorzugt aus einer Situation heraus, in welcher der erste Teilkreislauf ansonsten bereits gefüllt ist.

Insbesondere kann der erste Teilkreislauf in einem ersten Schritt befüllt werden, und die Befüllung der ersten Kammer des Membranfilter erst in einem zweiten Schritt erfolgen. Für den ersten Schritt kann der Membranfilter von dem ersten Teilkreislauf getrennt sein und erst für den zweiten Schritt fluidisch mit dem ersten Teilkreislauf verbunden werden.

Bevorzugt wird in einem ersten Schritt der erste Teilkreislauf befüllt, ohne dass die erste Kammer des Membranfilters fluidisch mit dem ersten Teilkreislauf in Verbindung steht, und in einem zweiten Schritt die erste Kammer des Membranfilters fluidisch mit dem ersten Teilkreislauf verbunden wird, um diese zu befüllen

Für den ersten Schritt kann der Membranfilter über Ventile von dem ersten Teilkreislauf getrennt und/oder durch eine Bypass-Leitung überbrückt werden. Nach der Befüllung des ersten Teilkreislaufs werden dann die Ventile geöffnet und/oder die Bypassleitung geschlossen.

Der erste Schritt kann ohne einen an der Blutbehandlungsvorrichtung angeordneten Membranfilter erfolgen, beispielsweise kann er am Ende einer vorhergehenden Behandlung erfolgen, beispielsweise um den ersten Teilkreislauf zu spülen. Bei der dann nachfolgenden Behandlung ist dann initial der erste Teilkreislauf mit Ausnahme des Membranfilters bereits gefüllt.

Alternativ kann für den ersten Schritt ein Kurzschlussstück an Stelle des Membranfilter in den ersten Teilkreislauf eingesetzt oder dessen Enden unmittelbar miteinander verbunden werden. Für den zweiten Schritt wird dann das Kurzschlussstück durch den Dialysator ersetzt und/oder der Dialysator in den ersten Teilkreislauf eingesetzt.

Die vorliegende Erfindung umfasst weiterhin eine Blutbehandlungsmaschine, an welche mindestens ein Membranfilter, insbesondere einen Hohlfasermembranfilter, mit einer ersten und einer zweiten Kammer, welche durch eine Membran semipermeabel getrennt sind, ankoppelbar ist, wobei die Blutbehandlungsmaschine umfasst:
- mindestens einen ersten Pumpaktor einer ersten Pumpe zum Pumpen von Flüssigkeit in einem ersten Teilkreislauf und mindestens einen zweiten Pumpaktor zum Pumpen von Flüssigkeit in einem zweiten Teilkreislauf, wobei die erste Kamer des Membranfilters im ersten Teilkreislauf und die zweite Kammer des Membranfilters im zweiten Teilkreislauf angeordnet ist, wobei die erste Pumpe stromaufwärts des Membranfilters in dem ersten Teilkreislauf angeordnet ist,
- mindestens zwei Drucksensoren zum Messen eines Drucks im ersten und eines Drucks im zweiten Teilkreislauf, und
- eine Steuerung, welche die Signale des Drucksensors auswertet und Aktoren der Blutbehandlungsmaschine ansteuert.

Die Blutbehandlungsmaschine ist dadurch gekennzeichnet, dass die Steuerung ein Füllprogramm umfasst, welches eine Ansteuerung des ersten Pumpaktors zur Befüllung der ersten Kammer des Membranfilters über den ersten Teilkreislauf in Abhängigkeit von dem im ersten Teilkreislauf gemessenen Druck und/oder dem Transmembrandruck vornimmt. Dabei ist vorgesehen, dass eine Förderrate der Pumpe bei der Befüllung verringert oder die Pumpe gestoppt wird, sobald der Druck im ersten Teilkreislauf und/oder der Transmembrandruck einen ersten Schwellwert übersteigt und die Förderrate der Pumpe bei der Befüllung erhöht oder die Pumpe wieder gestartet wird, sobald der Druck im ersten Teilkreislauf und/oder der Transmembrandruck einen zweiten Schwellwert unterschreitet. Hierdurch ergeben sich die gleichen Vorteile, welche bereits oben im Hinblick auf das erfindungsgemäße Verfahren näher beschrieben wurden.

Bei dem Membranfilter handelt es sich bevorzugt um ein Disposable, welches mit der Blutbehandlungsmaschine zur Durchführung einer Behandlung gekoppelt wird. Bei dem zweiten Teilkreislauf handelt es sich bevorzugt ebenfalls um ein Disposable.

Bei dem ersten Teilkreislauf kann es sich je nach Ausgestaltung der Blutbehandlungsmaschine um ein Disposable oder um einen festen Bestandteil der Blutbehandlungsmaschine handeln.

In einer möglichen Ausgestaltung der vorliegenden Erfindung steuert die Steuerung zur Durchführung des Füllprogramms einen oder mehrere Pumpaktoren und/oder einen oder mehrere Ventilaktoren, welche Ventile des ersten und/oder zweiten Teilkreislaufs betätigen, an.

In einer möglichen Ausgestaltung der vorliegenden Erfindung erfolgt die Abarbeitung des Füllprogramms automatisch durch die Steuerung.

In einer möglichen Ausgestaltung der vorliegenden Erfindung ist die Steuerung programmiert, um ein Verfahren, wie es oben näher beschrieben wurde, durchzuführen.

Bei der erfindungsgemäßen Blutbehandlungsmaschine kann es sich insbesondere um eine Dialysemaschine handeln, insbesondere zur Hämodialyse und/oder zur Hämofiltration und/oder zur Hämodiafiltration.

Die vorliegenden Erfindung umfasst weiterhin ein Blutbehandlungssystem aus einer Blutbehandlungsmaschine, wie sie oben beschrieben wurde, und einem Membranfilter. Das Blutbehandlungssystem umfasst bevorzugt weiterhin den ersten und/oder den zweiten Teilkreislauf.

Die vorliegenden Erfindung wird nun anhand von Ausführungsbeispielen und Figuren näher beschrieben.

Dabei zeigen:
- Fig. 1:: eine schematische, vereinfachte Darstellung der Fluidkreisläufe eines Ausführungsbeispiels eines erfindungsgemäßen Blutbehandlungssystems,
- Fig. 2:: ein Diagramm, in welchem der Verlauf des Transmembrandrucks während der Befüllung der ersten Kammer für ein Verfahren gemäß dem Stand der Technik und ein Verfahren gemäß der vorliegenden Erfindung gezeigt ist und
- Fig. 3: ein Diagramm, in welchem der Verlauf der von den einzelnen Drucksensoren des Blutbehandlungssystems gemessenen Drücke während der Befüllung der ersten Kammer bei einem Verfahren gemäß der vorliegenden Erfindung gezeigt ist.

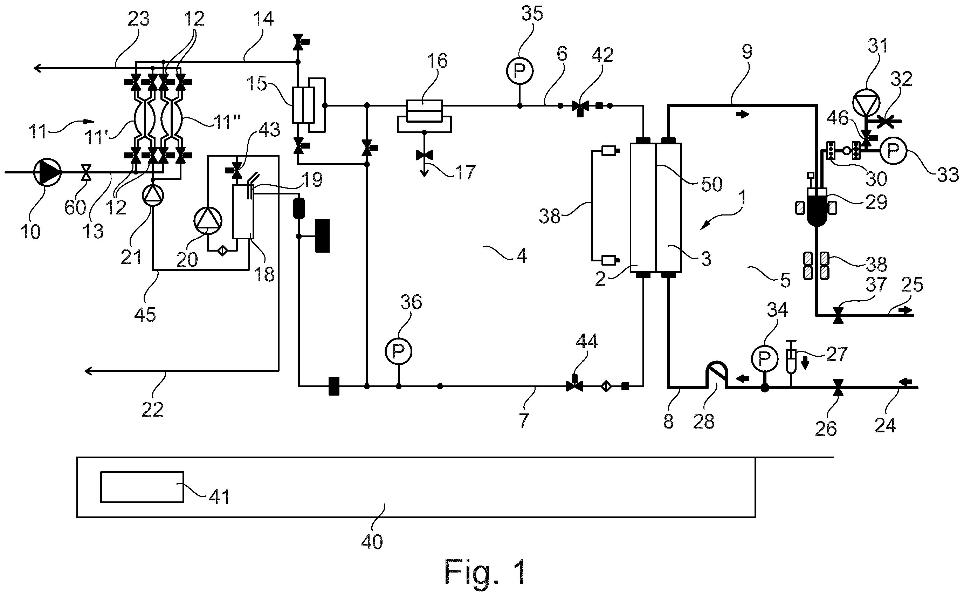

Fig. 1 zeigt eine schematische, vereinfachte Darstellung der Fluidkreisläufe eines Ausführungsbeispiels eines erfindungsgemäßen Blutbehandlungssystems. Dabei wurden von den üblicherweise vorhandenen Komponenten des Blutbehandlungssystems zumindest jene gezeigt, welche für die Durchführung der vorliegenden Erfindung beitragen können. Allerdings sind auch nicht alle gezeigten und beschriebenen Komponenten zur Durchführung der Erfindung zwingend notwendig. Dies ergibt sich aus der Beschreibung zur Funktion der einzelnen Komponenten.

Das Blutbehandlungssystem umfasst einen Membranfilter 1. Der Membranfilter 1 umfasst eine erste Kammer 2 und eine zweite Kammer 3, welche durch eine Membran 50 semipermeabel getrennt sind.

Das Blutbehandlungssystem umfasst weiterhin einen ersten Teilkreislauf 4 und einen zweiten Teilkreislauf 5. Dabei ist die erste Kammer 2 des Membranfilters 1 im ersten Teilkreislauf 4 angeordnet und die zweite Kammer 3 im zweiten Teilkreislauf 5. Die erste Kammer 2 des Membranfilters 1 steht eingangsseitig mit einer Zulaufleitung 6 und ausgangsseitig mit einer Ablaufleitung 7 des ersten Kreislaufs in Verbindung.

Im Ausführungsbeispiel handelt es sich bei dem Membranfilter 1 um einen Hohlfasermembranfilter, welcher als Dialysefilter ausgestaltet ist. Der erste Teilkreislauf 4 ist hierbei der Dialysatkreislauf, der zweite Teilkreislauf 5 der extrakorporale Blutkreislauf.

Das Blutbehandlungssystem umfasst weiterhin eine hier nur schematisch dargestellte Blutbehandlungsmaschine 40, welche mindestens einen ersten Pumpaktor einer ersten Pumpe 10 zum Pumpen von Flüssigkeit in dem ersten Teilkreislauf 4 und mindestens einen zweiten Pumpaktor einer Pumpe 28 zum Pumpen von Flüssigkeit in dem zweiten Teilkreislauf 5 umfasst. Die erste Pumpe 10 ist dabei stromaufwärts des Membranfilter 1 im Dialysatkreislauf angeordnet.

Weiterhin ist ein dritter Pumpaktor einer dritten Pumpe 21 sowie ein vierter Pumpaktor einer vierten Pumpe 20 um Pumpen von Flüssigkeit in dem ersten Teilkreislauf 4 vorgesehen, welche beide stromabwärts eines sekundären Luftabscheiders 18 im Dialysatkreislauf angeordnet sind.

Weiterhin sind Drucksensoren 35, 36 zum Messen eines Drucks im ersten Teilkreislauf und Drucksensoren 34, 33 zum Messen des Drucks im zweiten Teilkreislauf vorgesehen. Die Blutbehandlungsmaschine 40 weist eine ebenfalls nur schematisch dargestellte Steuerung 41 auf, welche die Signale der Drucksensoren 33-36 auswertet und die Aktoren der Blutbehandlungsmaschine ansteuert.

Der Membranfilter 1 und der zweite Teilkreislauf sind bevorzugt als Disposable ausgeführt und können zur Durchführung einer Behandlung an die Dialysemaschine 40 gekoppelt werden. Der erste Teilkreislauf kann je nach Ausgestaltung des Systems ebenfalls als Disposable ausgeführt sein oder zumindest teilweise oder ganz einen Teil der Blutbehandlungsmaschine 40 bilden.

Im Folgenden werden bevorzugte Merkmale des Blutbehandlungssystems näher beschrieben. Die vorliegende Erfindung kann jedoch auch mit anders aufgebauten Blutbehandlungssystemen verwirklicht werden

Im Ausführungsbeispiel bildet die erste Pumpe 10 im ersten Teilkreislauf 4 eine Ladepumpe, welche über die Leitung 13 eine Flüssigkeit, im Ausführungsbeispiel Dialysat, zu einer Bilanzkammeranordnung 11 pumpt. Von der Bilanzkammeranordnung 11 fließt die Flüssigkeit über die Leitung 14, 6 zum Eingang der ersten Kammer 2.

In der Leitung 14, 6 ist im Ausführungsbeispiel ist ein Sterilfilter 15 vorgesehen. Weiterhin ist stromabwärts des Sterilfilters 15 ein weiterer Sterilfilter 16 angeordnet, welcher eine zweite Filterstufe für die Substituatleitung 17 bildet.

Stromaufwärts der ersten Kammer 2 ist in der Zulaufleitung 6 ein Ventil 42 angeordnet, über welches der Zustrom zum Membranfilter 1 gesteuert werden kann. Weiterhin ist stromabwärts der ersten Kammer 2 in der Ablaufleitung 7 ein weiteres Ventil 44 vorgesehen.

Der Ausgang der ersten Kammer 2 steht über die Ablaufleitung 7 mit einem sekundären Luftabscheider 18 in Verbindung. Der sekundäre Luftabscheider weist einen Sensor 19 auf, über welchen Luft im sekundären Luftabscheider erkannt werden kann.

Eine Ablaufleitung 45 des Luftabscheiders 18 steht über die als Dialysatpumpe ausgestaltete dritte Pumpe 21 mit der Bilanzkammeranordnung 11 in Verbindung. Gebrauchtes Dialysat wird über die Dialysatpumpe 21 und die Bilanzkammeranordnung 11 in die Abflussleitung 23 gepumpt.

Weiterhin ist als vierte Pumpe eine Ultrafiltrationspumpe 20 vorgesehen, welche Dialysat von dem Luftabscheider 18 unter Umgehung der Bilanzkammeranordnung 11 unmittelbar zu einer Abflussleitung 22 pumpt.

Der sekundäre Luftabscheider 18 weist im Ausführungsbeispiel weiterhin in Entlüftungsventil 43 auf, welches den Luftabscheider 18 mit der Abflussleitung 22 verbindet.

Der zweite Teilkreislauf weist im Ausführungsbeispiel eine Zulaufleitung 8 auf, welche mit einem Eingang der zweiten Kammer 3 des Membranfilters 1 in Verbindung steht, sowie eine Ablaufleitung 9, welche mit dem Ausgang der zweiten Kammer 3 in Verbindung steht.

Im Ausführungsbeispiel ist die Zulaufleitung 8 Teil der arteriellen Leitung des extrakorporalen Blutkreislaufs 5, und die Ablaufleitung 9 Teil der venösen Leitung des extrakorporalen Blutkreislaufs. Die arterielle Leitung umfasst einen patientenseitigen Konnektor 24, eine arterielle Klemme 26, eine Heparinpumpe 27 sowie einen Pumpschlauchabschnitt, welcher in eine Rollenpumpe 28 einlegbar ist. Die venöse Leitung weist einen Blasenfänger 29, einen Blasendetektor 38, eine venöse Klemme 37 und einen patientenseitigen Konnektor 25 auf.

Am venösen Blasenfänger 29 ist über einen Filter 30, beispielsweise einen Hydrophobfilter und/oder einen Sterilfilter, ein Drucksensor 33 angeordnet. Weiterhin steht der Blasenfänger über den Filter 30 und die Drossel 32 weiterhin mit der Atmosphäre in Verbindung, wobei diese Verbindung durch ein Ventil 46 geöffnet und geschlossen werden kann. Weiterhin ist eine Luftpumpe 31 vorgesehen, durch welche ein Integritätstest der Membran 50 durchgeführt werden kann.

Im Ausführungsbeispiel sind Drucksensoren 35 und 36 vorgesehen, über welche der Druck stromaufwärts und stromabwärts der ersten Kammer des Membranfilters 1 im ersten Teilkreislauf 4 gemessen werden kann. Weiterhin ist ein Drucksensor 33 vorgesehen, um den Druck in der venösen Leitung des zweiten Teilkreislaufs zu messen, sowie ein Drucksensor 34, welcher den arteriellen Druck stromaufwärts der okkludierenden Rollenpumpe 28 misst.

Während der Blutbehandlung wird Dialysat durch die Wirkung der Ladepumpe 10, der Dialysatpumpe 21 und der Bilanzkammeranordnung 11 bilanzierend im ersten Teilkreislauf 4 bewegt. Dabei fließt das Dialysat von der Bilanzkammeranordnung 11 über den Sterilfilter 15 zur ersten Kammer 2 des Membranfilters 1 und von dort weiter über den sekundären Luftabscheider 19 und die Dialysatpumpe 21 zur Bilanzkammeranordnung 11. Im zweiten Teilkreislauf 5 wird das Blut von dem arteriellen Konnektor 24 über die Blutpumpe 28 zur zweiten Kammer gepumpt, durchströmt diese im Gegenstrom zum Dialysat, und fließt über den Blasenfänger 29 zum venösen Konnektor 25.

Vor Beginn der Behandlung müssen der erste Teilkreislauf 4, der zweite Teilkreislauf 5 sowie der Membranfilter 1 befüllt und optional gespült werden.

Ein Ausführungsbeispiel des erfindungsgemäßen Befüllverfahrens wird im Folgenden näher beschrieben:
Im Ausführungsbeispiel wird vor dem Befüllen des Membranfilters zunächst der erste Teilkreislauf 4 mit Flüssigkeit befüllt. Hierfür ist zwischen der Zulaufleitung 6 und der Ablaufleitung 7 des ersten Teilkreislaufs 4 an Stelle des Membranfilters 1 ein Kurzschlussstück 38 in den ersten Teilkreislauf eingefügt. Die Befüllung des ersten Teilkreislaufs 4 mit Flüssigkeit erfolgt beispielsweise durch Betätigung der Ladepumpe 10 zum Füllen einer Bilanzkammer der Bilanzkammeranordnung mit frischem Dialysat, das beispielsweise durch Verdrängung von Flüssigkeit, die mittels der Dialysatpumpe 21 gepumpt wird, aus dieser Bilanzkammer in die Leitung 14 verschoben wird. Alternativ kann auch mittels der Ladepumpe 10 Flüssigkeit aus der mit der Pumpe durch eine Ventilstellung nicht fluidisch verbundenen Bilanzkammerhälfte der ersten Bilanzkammer 11 in die zweite nicht fluidisch verbundene Bilanzkammerhälfte der zweiten Bilanzkammer 11' verschoben werden und aus der weiteren Bilanzkammerhälfte der zweiten Bilanzkammer 11' Flüssigkeit in die Leitung 14 überführt werden.

Nach der Befüllung des ersten Teilkreislaufs wird der noch unbefüllte Membranfilter 1 an Stelle des Kurzschlussstücks 38 in den ersten Teilkreislauf eingesetzt. Der zweite Teilkreislauf 5 ist ebenfalls mit dem Membranfilter 5 verbunden und wie dieser noch nicht mit Flüssigkeit befüllt.

Gemäß der vorliegenden Erfindung erfolgt nun eine druckgesteuerte Ansteuerung der ersten Pumpe 10 zur Befüllung der ersten Kammer 2 des Membranfilters 1 über den ersten Teilkreislauf 4 mit Flüssigkeit. Dabei wird der Druck im ersten und/oder zweiten Teilkreislauf gemessen und die Befüllung in Abhängigkeit von dem gemessenen Druck angesteuert. Die Ansteuerung der Befüllung erfolgt bevorzugt durch ein Füllprogramm der Steuerung 41 der Blutbehandlungsmaschine.

Das zentrale Merkmal des erfindungsgemäßen Vorgehens bzw. der erfindungsgemäßen Steuerung ist daher ein druckgesteuertes Füllprogramm zur Ansteuerung der ersten Pumpe 10, welches Lufteinschlüsse im Membranfilter vermeiden soll.

Wenn in dieser Beschreibung eine Ansteuerung, Starten und/oder Stoppen der Pumpe 10 genannt wird, so umfasst dies auch, dass die Pumpe selbst zwar weiterfördert, aber durch Öffnen oder Schließen eines oder mehrere Ventile, beispielsweise die Ventile der Bilanzkammern, ein Transport der Flüssigkeit beeinflusst, insbesondere freigegeben und/oder unterbunden wird.

Das Füllprogramm steuert die Befüllung bevorzugt in Abhängigkeit von dem Transmembrandruck über die Membran 50 des Membranfilters 1.

Bevorzugt wird die druckgesteuerte Befüllung fortgesetzt, solange eine Veränderung des venösen Druckes über den Drucksensor 33, und insbesondere Druckschwankungen, beobachtet wird.

Als optionale weitere Bedingung, die erfüllt sein muss, um die druckgesteuerte Befüllung zu beenden, kann gefordert werden, dass keine Luft am Sekundärluftabscheider mehr detektiert wird.

Daran kann sich eine Mindestrestfüllphase anschließen.

Merkmale eines bevorzugten Ausführungsbeispiels des Füllprogramms und des erfindungsgemäßen Verfahrens werden im folgenden beschrieben:
Im druckgesteuerten Füllprogramm macht die Blutbehandlungsmaschine bevorzugt Füllumschaltungen, d.h. die Ladepumpe 10 fördert Flüssigkeit in dem ersten Teilkreislauf 4 in Richtung auf den Membranfilter 1.

Hierfür weist die Bilanzkammeranordnung 11 zwei Bilanzkammern 11' und 11" auf, welche für die Füllumschaltungen über die zugeordneten Ventile 12 so verschaltet werden, dass kein bilanzierender Betrieb vorliegt, sondern die eine Bilanzkammer die andere antreibt.

Der Fluss kann bis zu einer vorgegebenen Umschaltung, im Ausführungsbeispiel bis zur 6. Umschaltung nach Anhängen des Membranfilters 1 auf eine erste, niedrige Flussrate begrenzt sein. Dies beträgt im Ausführungsbeispiel beispielsweise 500 ml/min. Nach der vorgegebenen Umschaltung kann die Füllpumpe 10 dagegen mit einer zweiten, höheren Flussrate arbeiten. Diese beträgt bevorzugt mehr als 1000 ml/min, bspw. 1200 ml/min.

Die Ladepumpe 10 kann dabei volumengesteuert arbeiten.

Überschreitet der Druck über die Membran 50 während dieses Befüllvorgangs jedoch einen ersten Schwellwert, wird die Umschaltung unterbrochen und/oder die Flussrate verringert, bis der Druck über die Membran wieder unter einen zweiten Schwellwert gefallen ist. Bevorzugt ist der zweite Schwellwert niedriger als der erste Schwellwert, um ein zu häufiges Schalten zu vermeiden.

Beispielsweise kann die Umschaltung unterbrochen und/oder die Flussrate verringert werden, wenn der durch Drucksensor 35 gemessene Druck am Eingang der ersten Kammer 2 den durch Drucksensor 33 gemessenen venösen Rückgabedruck um mehr als 230 mmHg übersteigt. Die Unterbrechung wird beigehalten, bis der Druckunterschied zum venösen Druck 200 mmHg wieder unterschreitet. Erst dann wird der normale Füllbetrieb fortgesetzt. Hierdurch wird der Druckunterschied über die Membran begrenzt.

Das Unterbrechen der Umschaltung der Bilanzkammer kann beispielsweise dadurch erfolgen, dass das zwischen der Ladepumpe 10 und der Bilanzkammeranordnung 11 angeordnete Frischwasserventil 60 geschlossen wird.

Je nach Ausgestaltung des Verfahrens kann das Ventil 43 geöffnet sein, so dass ein zweiter Strömungswegs zum Abführen der Luft aus der ersten Kammer 2 vorliegt, oder das Ventil 43 kann geschlossen sein. Bei einem geschlossenen Ventil wird der zum Aufbau eines zur Verdrängung von Luft über die Membran notwendigen Druckunterschieds über die Membran schneller erreicht. Dies kann die Füllung insbesondere großer Membranfilter beschleunigen.

Während des druckgesteuerten Füllprogramms kann vorgesehen sein, dass kein Flüssigkeitstransport von dem sekundären Luftabscheider 18 zur Abflussleitung 22 oder 23 erfolgt, d.h. die Pumpen 20 und 21 werden nicht betrieben und/oder die entsprechenden Ventile sind geschlossen.

Solange das druckgesteuerte Füllprogramm läuft, wird der Verlauf des venösen Drucks überwacht. Solange eine Veränderung und/oder Druckschwankungen des venösen Druckes oder Luft am sekundären Luftabscheider 18 beobachtet wird, verbleibt das Gerät im druckgesteuerten Füllprogramm, wie es zuvor beschrieben wurde. Eine Variation des venösen Drucks, welcher beispielsweise über den Drucksensor 33 gemessen wird, ist dabei ein Zeichen für die Luftabscheidung über die Membran 50 des Membranfilters 1 von der ersten Kammer 2 in die zweite Kammer 3. Luft im sekundären Luftabscheider 18 ist ein Zeichen, dass Luft aus der ersten Kammer 2 in die Ablaufleitung 7 verdrängt wird.

Werden weder Druckveränderungen, beispielsweise Druckschwankungen des venösen Drucks noch Luft im sekundären Luftabscheider 18 detektiert, wird der Membranfilter als komplett befüllt angesehen.

Das Füllprogramm sieht jedoch weiterhin eine Mindestanzahl von Umschaltungen über den Dialysator vor, nachdem eine komplette Befüllung detektiert wurde. Die Mindestanzahl von Umschaltungen wird bevorzugt ohne eine Drucksteuerung durchgeführt. Sollte während dieser Zeit erneut Luft erkannt werden, so wird diese über das Ventil 43 am Sekundärluftabschieder abgeschieden

Im folgenden wird das Ausführungsbeispiel des erfindungsgemäßen Verfahrens noch einmal mit weiteren Details beschrieben:
In einem ersten, optionalen Schritt wird der Membranfilter mit normalen, d.h. bilanzierenden Bilanzkammerumschaltungen gefüllt, bis im Sekundärluftabscheider 18 solange Luft erkannt wurde, dass man davon ausgehen muss, dass der Luftabscheider leer läuft (max. zum Beispiel 3 Umschaltungen mit durch Sensor 19 erkannter Luft). In einer möglichen Ausgestaltung der vorliegenden Erfindung kann auf diesen ersten Schritt jedoch auch verzichtet werden.

Erfindungsgemäß macht das Gerät dann Füllumschaltungen, d.h. nicht bilanzierende Umschaltungen, in dem erfindungsgemäßen druckgesteuerten Füllprogramm, d.h. die Ladepumpe 10 fördert in Richtung Membranfilter 1. Der Fluss ist beispielsweise auf 500 ml/min begrenzt.

Bei den Füllumschaltungen fördert die Ladepumpe mit voller oder reduzierter Leistung in Richtung Membranfilter. Die sich hieraus an der Membran ergebenden Druckspitzen sind zunächst durch die Compliance im System und die Strömungswiderstände begrenzt. Der an der Dialysatormembran erzeugte Überdruck zwischen der ersten Kammer und der zweiten Kammer sorgt dafür, dass Luft über die Membran 50 in die zweite Kammer übertritt und von dort über die Belüftung 32 in die Umgebung entweicht. Dies funktioniert solange, wie die Dialysatormembran noch an den Stellen, an welchen sich die Luftblasen befinden, unbenetzt und daher luftdurchlässig ist.

Überschreitet der Druck am Drucksensor 35 (Dialysatoreingang) den venösen Rückgabedruck jedoch um mehr als 230 mmHg, wird die Umschaltung unterbrochen, bis der Druckunterschied zum venösen Druck 200 mmHg wieder unterschreitet. Erst dann wird das Frischwasserventil 60 wieder geöffnet. Hierdurch wird der Druckunterschied über die Membran begrenzt.

Die Begrenzung des Flusses und/oder Druckes über die Membran stellt sicher, dass keine Luftpolster im Faserbündel eingeschlossen werden, die anschließend nicht mehr abgeschieden werden können.

In einer ersten Alternative ist Ventil 43 durchgehend geöffnet. Luft und Flüssigkeit aus der ersten Kammer 2 des Membranfilters 1 kann somit zusätzlich über diesen Weg zum Abfluss gelangen. Wieviel Luft über welchen Weg entweicht, hängt von den Strömungswiderständen der Membran, der Hydraulik und der Belüftung im zweiten Teilkreislauf ab.

Ventil 43 kann jedoch auch geschlossen werden, solange kein nennenswerter Dialysatausgangsdruck vorhanden ist, was die Füllung vor allem bei sehr großen Filtern beschleunigen könnte.

Solange das druckgesteuerte Füllprogramm läuft, wird der Verlauf des venösen Drucks überwacht. Solange eine Variation des venösen Druckes oder Luft am Sensor 19 beobachtet wird, verbleibt das Gerät im druckgesteuerten Füllprogramm, wie im vorherigen Schritt. Eine Variation des venösen Drucks ist dabei ein Zeichen, dass weiterhin Luft über die Membran verschoben wird.

Durch die obigen Überwachungen wird sichergestellt, dass die Membran am Ende dieses Schritts vollständig benetzt ist (oder, wenn die Membran z.B. nicht luftdurchlässig ist, keine Luft mehr über die Dialysatausgang entweicht), also der Membranfilter bestmöglich (ohne Drehen) gefüllt ist.

Im Anschluss an die Erfassung eines so befüllten Filters wird noch eine Mindestanzahl von Umschaltungen über den Membranfilter ausgeführt. Sollte während dieser Zeit erneut Luft erkannt werden, so wird diese über das druckgesteuerte Füllprogramm über Ventil 43 abgeschieden.

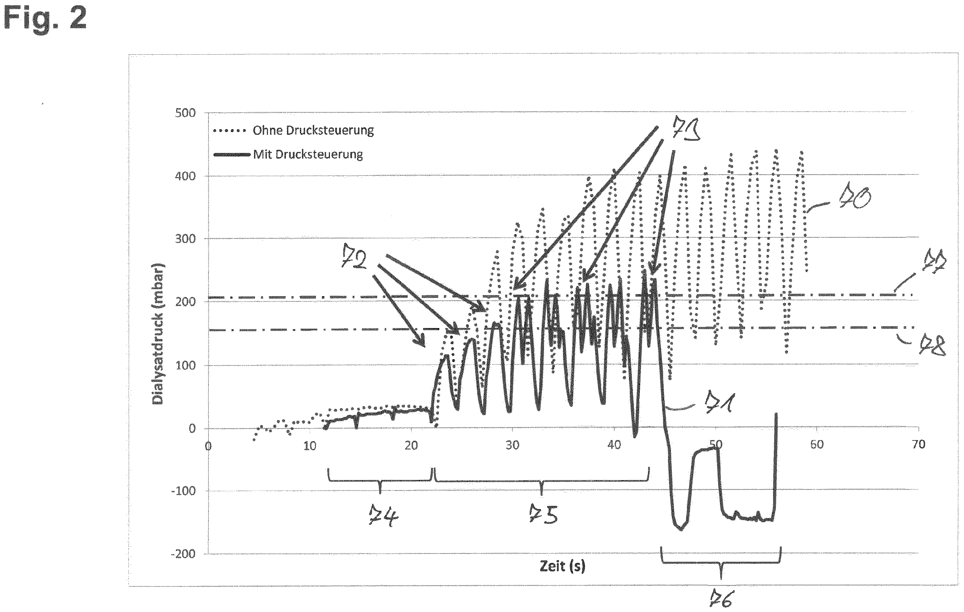

Fig. 2 zeigt den Verlauf des mittleren Dialysatdrucks bei einem Verfahren gemäß der vorliegenden Erfindung, d.h. mit Drucksteuerung, als Linie 71 im Vergleich zum Verlauf des mittleren Dialysatdrucks bei einem Verfahren gemäß dem Stand der Technik, d.h. ohne Drucksteuerung, welcher als Linie 70 dargestellt ist. Der mittlere Dialysatdruck wird als Mittelwert des Drucks am Eingang der Kammer 1 (Drucksensor 35) und am Ausgang der Kammer 1 (Drucksensor 36) berechnet.

Während der bilanzierenden Befüllphase 74, welche bei beiden Verfahren zum Einsatz kommt, liegt noch kein nennenswerter Dialysatdruck an, da die Flüssigkeit bzw. Luft lediglich im ersten Teilkreislauf verschoben wird. Gemäß der vorliegenden Erfindung kann auf diese bilanzierende Befüllphase 74 in einer alternativen Ausgestaltung jedoch auch verzichtet werden.

Im Rahmen der nicht-bilanzierenden, nunmehr druckgesteuerten Befüllphase 75 steigt der maximal erreichte Dialysatdruck zunächst mit jeder Bilanzkammerumschaltung an. In einer ersten Phase 72 erreichen die Druckspitzen noch nicht den ersten Schwellwert 77, so dass die Drucksteuerung nicht eingreift. Hier wird der Dialysatdruck daher allein durch die Compliance des Schlauchsets begrenzt, wie dies bereits gemäß dem Stand der Technik bekannt war.

In einer zweiten Phase 73 überschreiten die Druckspitzen dagegen den ersten Schwellwert 77. Wird dies durch die Steuerung erkannt, stoppt diese den Zufluss von Dialysat, bis der Transmembrandruck und/oder Dialysatdruck wieder unter einem zweiten, niedrigeren Schwellwert 78 liegt. Daraufhin wird die normale Befüllung wieder aufgenommen, bis der erste Schwellwert wieder überschritten wird. Im Ausführungsbeispiel ergeben sich daher pro Bilanzkammerumschaltung mehrere Druckspitzen.

Wie der Vergleich mit dem Stand der Technik unmittelbar ergibt, wird hierdurch mit einem erheblich niedrigeren maximalen Dialysatdruck als gemäß dem Stand der Technik gearbeitet. Die Druckbegrenzung verhindert, dass während der Befüllung der ersten Kammer Dialysat von der ersten Kammer in die zweite Kammer übertritt.

Nach dem Ende der druckgesteuerten Befüllphase 75 folgt eine Spülphase 76, in welcher der Druck wieder absinkt.

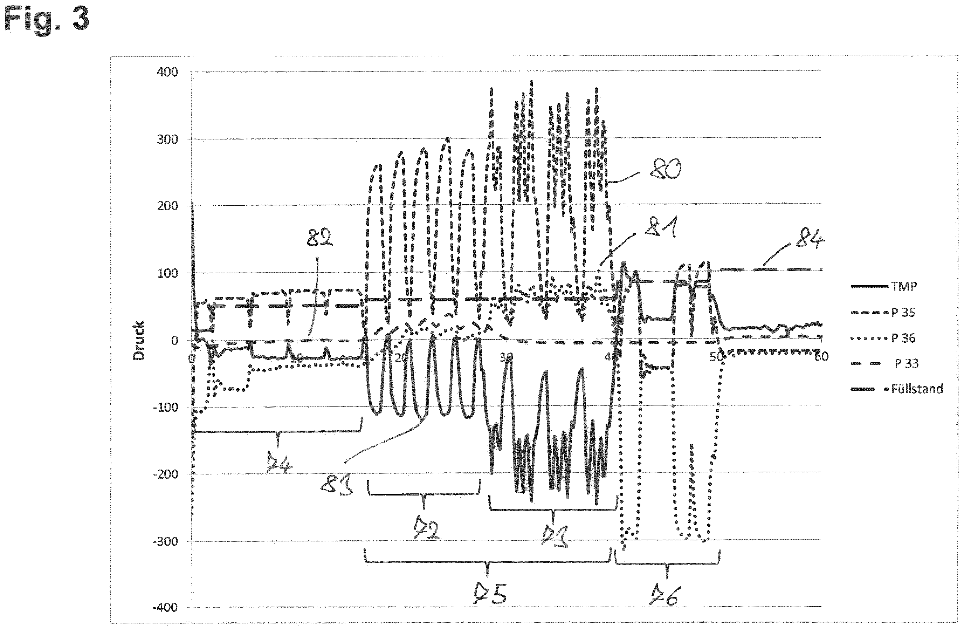

Fig. 3 zeigt nun detailliert die Druckverläufe an den einzelnen Drucksensoren während der soeben beschriebenen Phasen der Befüllung gemäß der vorliegenden Erfindung. Die einzelnen Linien zeigen folgendes:

| | |
|---|---|
| 80 | Druck am Drucksensor 35 (Druck am Eingang der ersten Kammer 2) |
| 81 | Druck am Drucksensor 36 (Druck am Ausgang der ersten Kammer 2) |
| 82 | Druck am Drucksensor 33 (Druck in der venösen Leitung/ der zweiten Kammer 3) |
| 83 | Transmembrandruck |
| 84 | Füllzustand der ersten Kammer |

Gut zu sehen ist, dass der Druck 82 in der venösen Leitung bzw. der zweiten Kammer 3 zu Beginn der druckgesteuerten Befüllphase 75 durch die über die Membran von der ersten Kammer in die zweite Kammer verdrängte Luft im Takt der Bilanzkammerumschaltungen schwankt. Nach einer gewissen Anzahl von Bilanzkammerumschaltungen verschwinden diese Druckschwankungen jedoch wieder, da nunmehr die Membran durchnässt ist und keine Luft mehr über die Membran verdrängt werden kann. Hierdurch steigt auch der Dialysatdruck 81 stromabwärts der ersten Kammer an.

Der Druck 82 auf der Blutseite kann erfindungsgemäß herangezogen werden, um die komplette Befüllung des Membranfilters bzw. die komplette Durchtränkung der Membran zu erfassen und/oder den zeitlichen Ablauf und/oder die Dauer des Befüllprogramms anzusteuern, wir dies oben näher beschrieben wurde. Insbesondere folgt auf die Erfassung des Endes der Druckschwankungen des Drucks 82 eine Mindestrestfüllphase, in welcher die erste Kammer weiter befüllt wird.

Der Transmembrandruck wird durch das druckgesteuerte Befüllprogramm auf einen Betrag, beispielsweise von 200 oder 250 mbar, begrenzt. Hierdurch wird insbesondere in der Mindestrestfüllphase, in welcher ohne eine solche Druckbegrenzung hohe Transmembrandrücke auftreten würden, ein Übertritt von Dialysat in die zweite Kammer verhindert.

Im Ausführungsbeispiel arbeitet das druckgesteuerte Befüllprogramm unmittelbar mit dem Transmembrandruck, welcher mit einem Schwellwert verglichen wird. In alternativen Ausgestaltungen könnte jedoch auch der mittlere Dialysatdruck und/oder der Druck am Eingang der ersten Kammer mit dem Schwellwert verglichen und begrenzt werden, da der Druck auf der Dialysatseite und insbesondere der Druck am Eingang der ersten Kammer ohnehin erheblich größer ist als der Druck auf der Blutseite und daher den Transmembrandruck stark bestimmt.

Nach dem Befüllen der ersten Kammer kann die zweite Kammer gemäß einem der aus dem Stand der Technik bekannten Verfahren befüllt werden. Die Befüllung kann beispielsweise durch Flüssigkeitsübertritt über die Membran 50 und/oder durch die Substituatleitung erfolgen.

## Patentansprüche

1. Verfahren zum Befüllen eines Membranfilters (1) eines Blutbehandlungssystems, wobei das Blutbehandlungssystem wenigstens eine Blutbehandlungsmaschine (40), einen Membranfilter (1), insbesondere einen Hohlfasermembranfilter, mit einer ersten und einer zweiten Kammer (2, 3), welche durch eine Membran (50) semipermeabel getrennt sind, und mindestens einen ersten Teilkreislauf (4) und mindestens einen zweiten Teilkreislauf (5) aufweist, wobei die erste Kammer (2) des Membranfilters (1) im ersten Teilkreislauf (4) und die zweite Kammer (3) des Membranfilters (1) im zweiten Teilkreislauf (5) angeordnet ist, wobei die Befüllung der ersten Kammer (2) des Membranfilters über den ersten Teilkreislauf (4) mit Flüssigkeit erfolgt, während die zweite Kammer (3) noch mit Luft gefüllt ist, wobei bevorzugt der erste Teilkreislauf (4) ein Dialysatkreislauf und/oder der zweite Teilkreislauf (5) ein extrakorporaler Blutkreislauf ist, und wobei stromaufwärts des Membranfilters (1) eine Pumpe (10) im ersten Teilkreislauf (4) angeordnet ist,
wobei die Ansteuerung der Pumpe (10) zur Befüllung der ersten Kammer (2) des Membranfilters (1) über den ersten Teilkreislauf (4) in Abhängigkeit von einem gemessenen Druck im ersten Teilkreislauf (4) und/oder von einem Transmembrandruck erfolgt, und
eine Förderrate der Pumpe (10) bei der Befüllung verringert oder die Pumpe (10) gestoppt wird, sobald der Druck im ersten Teilkreislauf (4) und/oder der Transmembrandruck einen ersten Schwellwert Z übersteigt, **dadurch gekennzeichnet, dass**
die Förderrate der Pumpe (10) bei der Befüllung erhöht oder die Pumpe (10) wieder gestartet wird, sobald der Druck im ersten Teilkreislauf (4) und/oder der Transmembrandruck einen zweiten Schwellwert unterschreitet.

2. Verfahren nach Anspruch 1, wobei der zur Befüllung eingesetzte, durch die Pumpe (10) erzeugte Volumenstrom und/oder der zeitliche Verlauf und/oder die Dauer der Befüllung durch die Pumpe (10) in Abhängigkeit von dem im ersten und/oder zweiten Teilkreislauf (4,5) gemessenen Druck angesteuert werden.

3. Verfahren nach Anspruch 1 oder 2, wobei ein Druck im ersten Teilkreislauf (4) und/oder ein Transmembrandruck über die Membran (50) des Membranfilters (1) bestimmt und die Ansteuerung der Pumpe (10) zur Befüllung der ersten Kammer (2) des Membranfilters (10) über den ersten Teilkreislauf (4) in Abhängigkeit von dem Druck im ersten Teilkreislauf (4) und/oder dem Transmembrandruck erfolgt.

4. Verfahren nach einem der vorangegangenen Ansprüche, wobei der erste Schwellwert insbesondere mindestens 50 mBar beträgt oder insbesondere mindestens 100 mBar beträgt, oder insbesondere mindestens 200 mBar beträgt, und/oder wobei der zweite Schwellwert insbesondere mindestens 50 mBar beträgt, oder insbesonderemindestens 100 mBar beträgt, oder insbesondere mindestens 150 mBar beträgt, und/oder wobei ein Absolutwert des ersten Schwellwerts größer ist als ein Absolutwert des zweiten Schwellwertes, und/oder wobei die Pumpe (10) so angesteuert wird, dass in der ersten Kammer (4) des Membranfilters (1) zumindest zeitweise ein Überdruck entsteht, insbesondere ein Druck von mindestens 50 mbar, oder insbesondere von mindestens 100 mBar, oder insbesondere von mindestens 200 mBar.

5. Verfahren nach einem der vorhergehenden Ansprüche, wobei die Pumpe (10) zur Befüllung der ersten Kammer (2) des Membranfilters (1) volumengesteuert angesteuert wird, wobei die volumengesteuerte Ansteuerung gestoppt oder der Volumenstrom verringert wird, sobald der Druck im ersten Teilkreislauf (4) und/oder der Transmembrandruck einen ersten Schwellwert überschreitet, wobei der erste Schwellwert insbesondere mindestens 50 mBar beträgt, oder insbesondere mindestens 100 mBar beträgt, oder insbesondere mindestens 200 mBar beträgt, und/oder wobei die volumengesteuerte Ansteuerung wiederaufgenommen oder der Volumenstrom vergrößert wird, sobald der Druck im ersten Teilkreislauf (4) und/oder der Transmembrandruck einen zweiten Schwellwert unterschreitet, wobei der zweite Schwellwert insbesondere mindestens 50 mBar beträgt, oder insbesondere mindestens 100 mBar beträgt, oder insbesondere mindestens 150 mBar beträgt.

6. Verfahren nach einem der vorhergehenden Ansprüche, wobei die erste Kammer (2) des Membranfilters (1) mit einem pulsatilen Volumenstrom befüllt wird, durch welchen in der ersten Kammer (2) des Membranfilters (1) Druckspitzen erzeugt werden, wobei die Pumpe bevorzugt mit einer Bilanzkammeranordnung (11) zusammenwirkt, welche durch die Pumpe (10) mit Flüssigkeit beaufschlagt wird und deren Umschaltungen zu einem pulsatilen Volumenstrom führen.

7. Verfahren nach einem der vorhergehenden Ansprüche, wobei während der Befüllung der ersten Kammer (2) des Membranfilters (1) zumindest zeitweise keine Flüssigkeit aus dem System abgeführt wird, insbesondere während der druckgesteuerten Befüllung, und/oder wobei während der Befüllung der ersten Kammer (2) des Membranfilters (1) eine stromabwärts des Membranfilters (1) im ersten Dialysatkreislauf angeordnete Pumpe (20, 21) zumindest zeitweise nicht betrieben wird.

8. Verfahren nach einem der vorhergehenden Ansprüche, wobei ein Druck im zweiten Teilkreislauf (5) gemessen und die Befüllung der ersten Kammer (2) des Membranfilters (1) über den ersten Teilkreislauf (2) in Abhängigkeit von dem Druck im zweiten Teilkreislauf (5) erfolgt, wobei bevorzugt dynamische und/oder statische Druckänderungen im zweiten Teilkreislauf (5) erfasst und/oder überwacht werden.

9. Verfahren nach Anspruch 8, wobei der Druck im zweiten Teilkreislauf (5) im Hinblick auf Druckänderungen, insbesondere Druckschwankungen, überwacht wird, wobei bevorzugt der zeitliche Ablauf der Befüllung von einer Detektion von den Druckänderungen, insbesondere den Druckschwankungen, abhängt und/oder wobei bevorzugt die komplette Befüllung der ersten Kammer (2) des Membranfilters (1) durch die Überwachung auf Druckänderungen, insbesondere Druckschwankungen erkannt wird, wobei bevorzugt auf eine komplette Befüllung geschlossen wird, wenn keine Druckänderungen, insbesondere Druckschwankungen mehr erfasst werden.

10. Verfahren nach einem der vorhergehenden Ansprüche, wobei die Befüllung so lange fortgesetzt wird, wie Druckänderungen, insbesondere Druckschwankungen im zweiten Teilkreislauf (5) und/oder Luft in einem sekundären Luftabscheider (18) des ersten Teilkreislaufs (4) erfasst werden, wobei bevorzugt noch eine Mindestrestfüllphase durchgeführt wird, nachdem keine Druckänderungen, insbesondere Druckschwankungen und/oder keine Luft mehr erfasst werden, wobei die Mindestrestfüllphase bevorzugt ein vorbestimmtes Pumpvolumen, eine vorbestimmte Zeit oder eine vorbestimmte Anzahl an Pumpschlägen und/oder Bilanzkammerumschaltungen umfasst.

11. Verfahren nach einem der vorhergehenden Ansprüche, wobei der zweite Teilkreislauf (4) während der Befüllung der ersten Kammer (2) des Membranfilters (1) über den ersten Teilkreislauf (4) in Fluidverbindung zur Atmosphäre steht, wobei bevorzugt der zweite Teilkreislauf (3) während der Befüllung der ersten Kammer (2) des Membranfilters (1) über ein Bauteil, das einen Druckabfall erzeugt, mit der Atmosphäre in Fluidverbindung steht, insbesondere über einen Filter (30) und/oder eine Drossel und/oder ein Ventil (46, 32).

12. Verfahren nach einem der vorhergehenden Ansprüche, wobei in einen Sekundärluftabscheider (18), welcher im ersten Teilkreislauf (4) stromabwärts des Membranfilters (1) angeordnet ist, während der Befüllung der ersten Kammer (2) des Membranfilters (1) über den ersten Teilkreislauf (4) Flüssigkeit und/oder Luft aus der ersten Kammer (2) verdrängt wird, wobei bevorzugt der Sekundärluftabscheider (18) zumindest zeitweise nicht mit einem Flüssigkeitsabfluss des Systems in Verbindung steht.

13. Verfahren nach einem der vorhergehenden Ansprüche, wobei der erste Teilkreislauf (4) während der druckgesteuerten Befüllung zumindest zeitweise nicht bilanzierend befüllt wird, wobei insbesondere die Befüllung der ersten Kammer (2) des Membranfilters (1) über den ersten Teilkreislaufs (4) ohne eine bilanzierende Phase startet und/oder zumindest bis zu einer Detektion einer Befüllung nicht-bilanzierend erfolgt.

14. Blutbehandlungsmaschine (40), an welche mindestens ein Membranfilter (1), insbesondere einen Hohlfasermembranfilter, mit einer ersten und einer zweiten Kammer (2,3), welche durch eine Membran (50) semipermeabel getrennt sind, ankoppelbar ist, wobei die Blutbehandlungsmaschine (40) umfasst:
- mindestens einen ersten Pumpaktor einer ersten Pumpe (10) zum Pumpen von Flüssigkeit in einem ersten Teilkreislauf (4), und mindestens einen zweiten Pumpaktor einer zweiten Pumpe (28) zum Pumpen von Flüssigkeit in einem zweiten Teilkreislauf (5), wobei die erste Kamer (2) des Membranfilters (1) im ersten Teilkreislauf (4) und die zweite Kammer (3) des Membranfilters (1) im zweiten Teilkreislauf (5) angeordnet ist, wobei die erste Pumpe (10) stromaufwärts des Membranfilter (1) in dem ersten Teilkreislauf (4) angeordnet ist,
- mindestens zweier Drucksensoren (34 - 36) zum Messen eines Drucks im ersten und im zweiten Teilkreislauf (4,5), und
- eine Steuerung (41), welche die Signale der Drucksensoren (34 - 36) auswertet und Aktoren der Blutbehandlungsmaschine (40) ansteuert,
wobei dass die Steuerung (41) ein Füllprogramm umfasst, welches eine Ansteuerung des ersten Pumpaktors (35, 36) zur Befüllung der ersten Kammer (2) des Membranfilters (1) über den ersten Teilkreislauf (4) in Abhängigkeit von den im ersten Teilkreislauf (4) gemessenen Druck und/oder von einem Transmembrandruck vornimmt,
wobei eine Förderrate der Pumpe (10) bei der Befüllung verringert oder die Pumpe (10) gestoppt wird, sobald der Druck im ersten Teilkreislauf (4) und/oder der Transmembrandruck einen ersten Schwellwert übersteigt, **dadurch gekennzeichnet, dass** die Förderrate der Pumpe (10) bei der Befüllung erhöht oder die Pumpe (10) wieder gestartet wird, sobald der Druck im ersten Teilkreislauf (4) und/oder der Transmembrandruck einen zweiten Schwellwert unterschreitet.

15. Blutbehandlungsmaschine nach Anspruch 14, wobei die Steuerung (41) zur Durchführung des Füllprogramms einen oder mehrere Pumpaktoren und/oder einen oder mehrere Ventilaktoren, welche Ventile des ersten und/oder zweiten Teilkreislaufs (4,5) betätigen, ansteuert und/oder wobei die Abarbeitung des Füllprogramms automatisch durch die Steuerung (41) erfolgt, und/oder wobei die Steuerung (41) programmiert ist, ein Verfahren nach einem der Ansprüche 1 bis 13 durchzuführen.

## Claims

1. Method of filling a membrane filter (1) of a blood treatment system, wherein the blood treatment system comprises at least one blood treatment machine (40), a membrane filter (1), in particular a hollow-fiber membrane filter, having a first and a second chamber (2, 3) which are separated by a membrane (50) in a semipermeable manner, and at least one first partial circuit (4) and at least one second partial circuit (5), wherein the first chamber (2) of the membrane filter (1) is arranged in the first partial circuit (4) and the second chamber (3) of the membrane filter (1) is arranged in the second partial circuit (5), wherein the filling of the first chamber (2) of the membrane filter with liquid via the first partial circuit (4) takes place while the second chamber (3) is still filled with air, wherein the first partial circuit (4) is preferably a dialyzate circuit and/or the second partial circuit (5) is an extracorporeal blood circuit, and wherein a pump (10) is arranged upstream of the membrane filter (1) in the first partial circuit (4),
wherein the control of the pump (10) for filling the first chamber (2) of the membrane filter (1) via the first partial circuit (4) takes place depending on a measured pressure in the first partial circuit (4) and/or on a transmembrane pressure, and a conveying rate of the pump (10) during the filling process is reduced or the pump (10) is stopped as soon as the pressure in the first partial circuit (4) and/or the transmembrane pressure exceeds/exceed a first threshold value,
**characterized in that**
the conveying rate of the pump (10) during the filling process is increased or the pump (10) is started again as soon as the pressure in the first partial circuit (4) and/or the transmembrane pressure falls/fall below a second threshold value.

2. Method in accordance with claim 1, wherein the volume flow used for filling and generated by the pump (10) and/or the time development and/or the duration of the filling by the pump (10) are controlled depending on the pressure measured in the first and/or second partial circuit(s) (4, 5).

3. Method in accordance with claim 1 or 2, wherein a pressure in the first partial circuit (4) and/or a transmembrane pressure across the membrane (50) of the membrane filter (1) is determined and the control of the pump (10) for filling the first chamber (2) of the membrane filter (10) via the first partial circuit (4) takes place depending on the pressure in the first partial circuit (4) and/or on the transmembrane pressure.

4. Method in accordance with any one of the preceding claims, wherein the first threshold value in particular amounts to at least 50 mbar, or in particular amounts to at least 100 mbar, or in particular amounts to at least 200 mbar, and/or wherein the second threshold value in particular amounts to at least 50 mbar, or in particular amounting to at least 100 mbar or in particular amounts to at least 150 mbar, and/or wherein an absolute value of the first threshold value is greater than an absolute value of the second threshold value and/or wherein the pump (10) is controlled such that an excess pressure arises, at least at times, in the first chamber (4) of the membrane filter (1), in particular a pressure of at least 50 mbar, or in particular of at least 100 mbar, or in particular of at least 200 mbar.

5. Method in accordance with any one of the preceding claims, wherein the pump (10) for filling the first chamber (2) of the membrane filter (1) is controlled in a volume-controlled manner, wherein the volume-controlled control is stopped or the volume flow is reduced as soon as the pressure in the first partial circuit (4) and/or the transmembrane pressure exceeds/exceed a first threshold value, wherein the first threshold value in particular amounts to at least 50 mbar, or in particular amounts to at least 100 mbar, or in particular amounts to at least 200 mbar, and/or wherein the volume-controlled control is resumed or the volume flow is increased as soon as the pressure in the first partial circuit (4) and/or the transmembrane pressure falls below a second threshold value, wherein the second threshold value in particular amounts to at least 50 mbar, or in particular amounts to at least 100 mbar, or in particular amounts to at least 150 mbar.

6. Method in accordance with any one of the preceding claims, wherein the first chamber (2) of the membrane filter (1) is filled with a pulsatile volume flow by which pressure peaks are generated in the first chamber (2) of the membrane filter (1), wherein the pump preferably cooperates with a balancing chamber assembly (11) that has liquid applied by the pump (10) and whose switching results in a pulsatile volume flow.

7. Method in accordance with any one of the preceding claims, wherein no liquid is drained from the system, at least at times, during the filling process of the first chamber (2) of the membrane filter (1), in particular during the pressure-controlled filling process; and/or wherein a pump (20, 21) arranged downstream of the membrane filter (1) in the first dialyzate circuit is not operated, at least at times, during the filling process of the first chamber (2) of the membrane filter (1).

8. Method in accordance with any one of the preceding claims, wherein a pressure in the second partial circuit (5) is measured and the filling of the first chamber (2) of the membrane filter (1) takes place via the first partial circuit (2) depending on the pressure in the second partial circuit (5), wherein dynamic and/or static pressure changes in the second partial circuit (5) are preferably detected and/or monitored.

9. Method in accordance with claim 8, wherein the pressure in the second partial circuit (5) is monitored for pressure changes, in particular pressure fluctuations, wherein the time sequence of the filling in particular depends on a detection of the pressure changes, in particular the pressure fluctuations, and/or wherein the complete filling of the first chamber (2) of the membrane filter (1) is preferably recognized by the monitoring for pressure changes, in particular pressure fluctuations, wherein a conclusion is preferably drawn on a complete filling when pressure changes, in particular pressure fluctuations, are no longer detected.

10. Method in accordance with any one of the preceding claims, wherein the filling is continued for as long as pressure changes, in particular pressure fluctuations, are detected in the second partial circuit (5) and/or air is detected in a secondary air separator (18) of the first partial circuit (4), wherein a minimum residual filling phase preferably is still carried out once pressure changes, in particular pressure fluctuations, and/or air are no longer detected, wherein the minimum residual filling phase preferably comprises a predefined pump volume, a predefined time, or a predefined number of pump beats and/or balancing chamber switchovers.

11. Method in accordance with any one of the preceding claims, wherein the second partial circuit (4) is in fluid communication with the atmosphere during the filling process of the first chamber (2) of the membrane filter (1) via the first partial circuit (4), wherein preferably the second partial circuit (3) is in fluid communication with the atmosphere during the filling process of the first chamber (2) of the membrane filter (1) via an element that generates a pressure drop, in particular via a filter (30) and/or a restrictor and/or a valve (46, 32).

12. Method in accordance with any one of the preceding claims, wherein liquid and/or air is/are displaced from the first chamber (2) during the filling process of the first chamber of the membrane filter (1) via the first partial circuit in a secondary air separator (18) that is arranged in the first partial circuit (4) downstream of the membrane filter (1), wherein the secondary air separator (18) is preferably not connected to a liquid outflow of the system at least at times.

13. Method in accordance with any one of the preceding claims, wherein the first partial circuit (4) is not filled in a balancing manner, at least at times, during the pressure-controlled filling process, wherein the filling of the first chamber (2) of the membrane filter (1) via the first partial circuit (4) in particular starts without a balancing phase and/or takes place in a non-balancing manner at least until filling is detected.

14. Blood treatment machine (40), to which at least one membrane filter (1), in particular a hollow-fiber membrane filter, can be coupled, having a first chamber and a second chamber (2, 3) which are separated by a membrane (50) in a semipermeable manner, wherein the blood treatment machine (40) comprises:
- at least one first pump actuator of a first pump (10) for pumping liquid in a first partial circuit (4) and at least one second pump actuator of a second pump (28) for pumping liquid in a second partial circuit (5), wherein the first chamber (2) of the membrane filter (1) is arranged in the first partial circuit (4) and the second chamber (3) of the membrane filter (1) is arranged in the second partial circuit (5), wherein the first pump (10) is arranged upstream of the membrane filter (1) in the first partial circuit (4);
- at least two pressure sensors (34 - 36) for measuring a pressure in the first and second partial circuit (4, 5); and
- a control unit (41) which evaluates the signals of the pressure sensors (34 - 36) and controls actuators of the blood treatment machine (40), wherein the control unit (41) comprises a filling program that carries out a control of the first pump actuator (35, 36) for filling the first chamber (2) of the membrane filter (1) via the first partial circuit (4) depending on the pressures measured in the first partial circuit (4) and/or a transmembrane pressure, wherein a conveying rate of the pump (10) during the filling process is reduced or the pump (10) is stopped as soon as the pressure in the first partial circuit (4) and/or the transmembrane pressure exceeds/exceed a first threshold value,
**characterized in that**
the conveying rate of the pump (10) during the filling process is increased or the pump (10) is started again as soon as the pressure in the first partial circuit (4) and/or the transmembrane pressure falls/fall below a second threshold value.

15. Blood treatment machine in accordance with claim 14, wherein the control unit (41) controls one or more pump actuators and/or one or more valve actuators that actuate valves of the first and/or second partial circuit(s) (4, 5) for the carrying out of the filling program; and/or wherein the working through of the filling program takes place automatically by the control unit (41) and/or wherein the control unit (41) is programmed to perform a method according to any one of claims 1 to 13.

## Revendications

1. Procédé de remplissage d'un filtre à membrane (1) d'un système de traitement du sang, le système de traitement du sang présentant au moins une machine de traitement du sang (40), un filtre à membrane (1), notamment un filtre à membrane à fibres creuses, avec une première et une deuxième chambre (2, 3), qui sont séparées de manière semi-perméable par une membrane (50), et au moins un premier circuit partiel (4) et au moins un deuxième circuit partiel (5), la première chambre (2) du filtre à membrane (1) étant agencée dans le premier circuit partiel (4) et la deuxième chambre (3) du filtre à membrane (1) dans le deuxième circuit partiel (5), le remplissage de la première chambre (2) du filtre à membrane avec du liquide s'effectuant par l'intermédiaire du premier circuit partiel (4), tandis que la deuxième chambre (3) est encore remplie d'air, le premier circuit partiel (4) étant de préférence un circuit de dialysat et/ou le deuxième circuit partiel (5) étant un circuit sanguin extracorporel, et une pompe (10) étant agencée en amont du filtre à membrane (1) dans le premier circuit partiel (4),
la commande de la pompe (10) pour le remplissage de la première chambre (2) du filtre à membrane (1) par l'intermédiaire du premier circuit partiel (4) s'effectuant en fonction d'une pression mesurée dans le premier circuit partiel (4) et/ou d'une pression transmembranaire, et un débit de la pompe (10) étant réduit lors du remplissage ou la pompe (10) étant arrêtée dès que la pression dans le premier circuit partiel (4) et/ou la pression transmembranaire dépasse une première valeur seuil,
**caractérisé en ce que**
le débit de la pompe (10) est augmenté lors du remplissage ou la pompe (10) est redémarrée dès que la pression dans le premier circuit partiel (4) et/ou la pression transmembranaire passe en dessous d'une deuxième valeur seuil.

2. Procédé selon la revendication 1, le débit volumique utilisé pour le remplissage, généré par la pompe (10), et/ou l'évolution temporelle et/ou la durée du remplissage étant commandés par la pompe (10) en fonction de la pression mesurée dans le premier et/ou le deuxième circuit partiel (4, 5).

3. Procédé selon la revendication 1 ou 2, une pression dans le premier circuit partiel (4) et/ou une pression transmembranaire étant déterminée par l'intermédiaire de la membrane (50) du filtre à membrane (1) et la commande de la pompe (10) pour le remplissage de la première chambre (2) du filtre à membrane (10) par l'intermédiaire du premier circuit partiel (4) étant effectuée en fonction de la pression dans le premier circuit partiel (4) et/ou de la pression transmembranaire.

4. Procédé selon l'une quelconque des revendications précédentes, la première valeur seuil étant notamment d'au moins 50 mbar, ou notamment d'au moins 100 mbar, ou notamment d'au moins 200 mbar, et/ou la deuxième valeur seuil étant notamment d'au moins 50 mbar, ou notamment d'au moins 100 mbar, ou notamment d'au moins 150 mbar, et/ou une valeur absolue de la première valeur seuil étant supérieure à une valeur absolue de la deuxième valeur seuil, et/ou la pompe (10) étant commandée de telle sorte qu'une surpression se forme au moins temporairement dans la première chambre (4) du filtre à membrane (1), notamment une pression d'au moins 50 mbar, ou notamment d'au moins 100 mbar, ou notamment d'au moins 200 mbar.

5. Procédé selon l'une quelconque des revendications précédentes, la pompe (10) pour le remplissage de la première chambre (2) du filtre à membrane (1) étant commandée en volume, la commande en volume étant arrêtée ou le débit volumique étant réduit dès que la pression dans le premier circuit partiel (4) et/ou la pression transmembranaire dépasse une première valeur seuil, la première valeur seuil étant notamment d'au moins 50 mbar, ou notamment d'au moins 100 mbar, ou notamment d'au moins 200 mbar, et/ou la commande en volume étant reprise ou le débit volumique étant augmenté dès que la pression dans le premier circuit partiel (4) et/ou la pression transmembranaire passe en dessous d'une deuxième valeur seuil, la deuxième valeur seuil étant notamment d'au moins 50 mbar, ou notamment d'au moins 100 mbar, ou notamment d'au moins 150 mbar.

6. Procédé selon l'une quelconque des revendications précédentes, la première chambre (2) du filtre à membrane (1) étant remplie avec un débit volumique pulsatoire qui génère des pics de pression dans la première chambre (2) du filtre à membrane (1), la pompe coopérant de préférence avec un agencement de chambre d'équilibrage (11) qui est alimenté en liquide par la pompe (10) et dont les commutations conduisent à un débit volumique pulsatoire.

7. Procédé selon l'une quelconque des revendications précédentes, aucun liquide n'étant évacué du système au moins temporairement pendant le remplissage de la première chambre (2) du filtre à membrane (1), notamment pendant le remplissage commandé par la pression, et/ou une pompe (20, 21) agencée en aval du filtre à membrane (1) dans le premier circuit de dialysat ne fonctionnant pas au moins temporairement pendant le remplissage de la première chambre (2) du filtre à membrane (1).

8. Procédé selon l'une quelconque des revendications précédentes, une pression étant mesurée dans le deuxième circuit partiel (5) et le remplissage de la première chambre (2) du filtre à membrane (1) étant effectué par l'intermédiaire du premier circuit partiel (2) en fonction de la pression dans le deuxième circuit partiel (5), des variations de pression dynamiques et/ou statiques dans le deuxième circuit partiel (5) étant de préférence détectées et/ou surveillées.

9. Procédé selon la revendication 8, la pression dans le deuxième circuit partiel (5) étant surveillée en ce qui concerne les variations de pression, notamment les fluctuations de pression, le déroulement temporel du remplissage dépendant de préférence d'une détection des variations de pression, notamment des fluctuations de pression, et/ou le remplissage complet de la première chambre (2) du filtre à membrane (1) étant de préférence détecté par la surveillance des variations de pression, notamment des fluctuations de pression, à un remplissage complet étant de préférence constaté lorsqu'on ne détecte plus de variations de pression, notamment de fluctuations de pression.

10. Procédé selon l'une quelconque des revendications précédentes, le remplissage se poursuivant tant que des variations de pression, notamment des fluctuations de pression dans le deuxième circuit partiel (5) et/ou de l'air dans un séparateur d'air secondaire (18) du premier circuit partiel (4) sont détectés, une phase de remplissage restante minimale étant de préférence encore réalisée après qu'aucune variation de pression, notamment fluctuation de pression et/ou qu'aucun air n'est plus détecté, la phase de remplissage restante minimale comprenant de préférence un volume de pompage prédéterminé, un temps prédéterminé ou un nombre prédéterminé de coups de pompe et/ou de commutations de chambre d'équilibrage.

11. Procédé selon l'une quelconque des revendications précédentes, le deuxième circuit partiel (4) étant en communication fluidique avec l'atmosphère pendant le remplissage de la première chambre (2) du filtre à membrane (1) par l'intermédiaire du premier circuit partiel (4), le deuxième circuit partiel (3) étant de préférence en communication fluidique avec l'atmosphère pendant le remplissage de la première chambre (2) du filtre à membrane (1) par l'intermédiaire d'un composant générant une chute de pression, notamment par l'intermédiaire d'un filtre (30) et/ou d'un étranglement et/ou d'une vanne (46, 32).

12. Procédé selon l'une quelconque des revendications précédentes, du liquide et/ou de l'air étant déplacé hors de la première chambre (2) pendant le remplissage de la première chambre (2) du filtre à membrane (1) par l'intermédiaire du premier circuit partiel (4) dans un séparateur d'air secondaire (18) qui est agencé dans le premier circuit partiel (4) en aval du filtre à membrane (1), le séparateur d'air secondaire (18) n'étant de préférence pas en communication au moins temporairement avec une sortie de liquide du système.

13. Procédé selon l'une quelconque des revendications précédentes, le premier circuit partiel (4) étant rempli au moins temporairement de manière non équilibrante pendant le remplissage commandé par la pression, notamment le remplissage de la première chambre (2) du filtre à membrane (1) par l'intermédiaire du premier circuit partiel (4) démarrant sans une phase équilibrante et/ou s'effectuant de manière non équilibrante au moins jusqu'à la détection d'un remplissage.

14. Machine de traitement du sang (40), à laquelle peut être couplé au moins un filtre à membrane (1), notamment un filtre à membrane à fibres creuses, avec une première et une deuxième chambre (2, 3), qui sont séparées de manière semi-perméable par une membrane (50), la machine de traitement du sang (40) comprenant :
- au moins un premier actionneur de pompage d'une première pompe (10) pour pomper du liquide dans un premier circuit partiel (4), et au moins un deuxième actionneur de pompage d'une deuxième pompe (28) pour pomper du liquide dans un deuxième circuit partiel (5), la première chambre (2) du filtre à membrane (1) étant agencée dans le premier circuit partiel (4) et la deuxième chambre (3) du filtre à membrane (1) étant agencée dans le deuxième circuit partiel (5), la première pompe (10) étant agencée en amont du filtre à membrane (1) dans le premier circuit partiel (4),
- au moins deux capteurs de pression (34 - 36) pour mesurer une pression dans le premier et le deuxième circuit partiel (4, 5), et
- une commande (41) qui évalue les signaux des capteurs de pression (34 - 36) et qui commande des actionneurs de la machine de traitement du sang (40), la commande (41) comprenant un programme de remplissage, qui procède à une commande du premier actionneur de pompe (35, 36) pour le remplissage de la première chambre (2) du filtre à membrane (1) par l'intermédiaire du premier circuit partiel (4) en fonction de la pression mesurée dans le premier circuit partiel (4) et/ou d'une pression transmembranaire, un débit de la pompe (10) étant réduit lors du remplissage ou la pompe (10) étant arrêtée dès que la pression dans le premier circuit partiel (4) et/ou la pression transmembranaire dépasse une première valeur seuil,
**caractérisée en ce que**
le débit de la pompe (10) est augmenté lors du remplissage ou la pompe (10) est redémarrée dès que la pression dans le premier circuit partiel (4) et/ou la pression transmembranaire passe en dessous d'une deuxième valeur seuil.

15. Machine de traitement du sang selon la revendication 14, la commande (41) commandant pour l'exécution du programme de remplissage un ou plusieurs actionneurs de pompe et/ou un ou plusieurs actionneurs de soupape qui actionnent des soupapes du premier et/ou du deuxième circuit partiel (4, 5), et/ou la réalisation du programme de remplissage étant effectuée automatiquement par la commande (41), et/ou la commande (41) étant programmée pour exécuter un procédé selon l'une quelconque des revendications 1 à 13.
